# EUROPÄISCHE PATENTSCHRIFT

(11) **EP 4 517 276 B1**
(45) Veröffentlichungstag und Bekanntmachung des Hinweises auf die Patenterteilung: **22.04.2026**
(21) Anmeldenummer: 24196724.9
(22) Anmeldetag: 27.08.2024
(51) Int. Cl.: G01F 1/58, G01F 1/74

(54) **VERFAHREN ZUR BESTIMMUNG DES VOLUMENANTEILS EINER PHASE EINES MEHRPHASIGEN MEDIUMS UND ENTSPRECHENDES MESSGERÄT**
METHOD FOR DETERMINING THE VOLUME FRACTION OF A PHASE OF A MULTI-PHASE MEDIUM AND CORRESPONDING MEASURING INSTRUMENT
PROCEDE DE DETERMINATION DE LA PROPORTION VOLUMIQUE D'UNE PHASE D'UN MILIEU MULTIPHASE ET DISPOSITIF DE MESURE CORRESPONDANT

(30) Priorität: 29.08.2023 DE 102023123216
(43) Veröffentlichungstag der Anmeldung: 05.03.2025
(73) Patentinhaber: Krohne Messtechnik GmbH, 47058 Duisburg (DE)
(72) Erfinder: Förster, Herr Jan, 45149 Essen (DE); Krawczyk-Becker, Martin, 44627 Herne (DE); Wacker, Paul, 47059 Duisburg (DE)
(74) Vertreter: Gesthuysen Patentanwälte

(56) Entgegenhaltungen:
- US-A1- 2008 016 967
- US-A1- 2020 080 877
- US-B2- 8 340 920

## Beschreibung

Die Patentanmeldung US 2020/0080877 A1 beschreibt einen magnetischen Durchflussmesser mit Elektrodensensoren, bei dem ein Modul zur Identifizierung von Verunreinigungen den Rauschpegel im Sensorsignal verwendet, um festzustellen, ob sich eine Verunreinigung in der Leitung befindet, gemäß dem Stand der Technik der vorliegenden Erfindung.

Die Erfindung betrifft ein Verfahren zur Bestimmung des Volumenanteils einer Phase eines mehrphasigen Mediums gemäß Anspruch 1, wobei das Medium eine erste Phase mit einem ersten Wert für einen ersten elektrischen Parameter und zumindest zeitweilig eine zweite Phase mit einem zweiten Wert für den ersten elektrischen Parameter aufweist, wobei das Medium über ein Elektrodenpaar mit einer Mehrzahl elektrischer Anregungssignale beaufschlagt wird und zu den elektrischen Anregungssignalen eine Mehrzahl entsprechender elektrischer Reaktionssignale erfasst wird, wobei aus der Mehrzahl an Anregungssignalen und der Mehrzahl an Reaktionssignalen eine Mehrzahl von Werten für den ersten elektrischen Parameter des Mediums ermittelt wird. Ferner betrifft die Erfindung auch ein Messgerät mit einem zur Aufnahme eines Mediums dienenden Messvolumens gemäß Anspruch 13, , wobei das Medium eine erste Phase mit einem ersten Wert für einen ersten elektrischen Parameter und zumindest zeitweilig eine zweite Phase mit einem zweiten Wert für den ersten elektrischen Parameter aufweist, mit einer Steuer- und Auswerteeinheit, mit mit dem Medium in Kontakt stehenden Elektroden, wobei die Steuer- und Auswerteeinheit im Betriebszustand des Messgeräts das Medium über das Elektrodenpaar mit einer Mehrzahl elektrischer Anregungssignale beaufschlagt und zu den elektrischen Anregungssignalen eine Mehrzahl entsprechender elektrischer Reaktionssignale erfasst, wobei aus der Mehrzahl an Anregungssignalen und der Mehrzahl an Reaktionssignalen eine Mehrzahl von Werten für den ersten elektrischen Parameter des Mediums ermittelt wird.

Verfahren und Messgeräte der vorgenannten Art finden oft in Zusammenhang mit anderen Messverfahren Anwendung, wie beispielsweise bei der Durchflussmessung. Bei der Durchflussmessung wird - auch mittelbar über die Fließgeschwindigkeit des Mediums - ein Volumenstrom berechnet (beispielsweise mit magnetisch-induktiven Durchflussmessgeräten) oder auch ein Massestrom (beispielsweise mit Coriolis-Massedurchflussmessgeräten). Wenn bekannt ist, dass bei der Messaufgabe das Medium mehrere Phasen aufweisen kann, dann ist es einleuchtend, dass nicht nur der Volumenstrom oder der Massestrom des Mehrphasenmediums von Interesse ist, sondern auch die Zusammensetzung des Mediums also die Anteile der verschiedenen Phasen an dem Mehrphasenmedium. Der Begriff Phase ist hier also nicht einschränkend im Sinne eines Aggregatszustandes zu verstehen, sondern es ist darunter weiter ein Mehrstoffsystem zu verstehen. Solche Mehrstoffsysteme liegen bei vielen Anwendungen in der chemischen Industrie, bei der Ölund Gasgewinnung, der Abwasserverarbeitung usw. vor.

Voraussetzung für die Durchführung des Verfahrens ist das Vorhandensein von Elektroden, die mit dem Medium in Kontakt stehen und über die das Medium mit einem elektrischen Anregungssignal beaufschlagt werden kann. Es ist beispielsweise bekannt, dass Medium mit einer elektrischen Spannung als elektrisches Anregungssignal zu beaufschlagen und als elektrisches Reaktionssignal den verursachten elektrischen Strom zu erfassen. Aus den Werten der elektrischen Spannung und des elektrischen Stromes können dann Werte für den ohmschen Widerstand oder die Impedanz des Mediums als erster elektrischer Parameter des Mediums ermittelt werden. Wenn davon ausgegangen wird, dass sich der erste elektrische Parameter der ersten Phase in Reinform und der zweiten Phase in Reinform voneinander unterscheiden, dann ist es einleuchtend, dass sich auch der erste elektrische Parameter des mehrphasigen Mediums mit sich ändernden Anteilen der ersten Phase und der zweiten Phase ändert und der Anteil der zweiten Phase (und damit auch der ersten Phase) über den Zusammenhang zwischen dem ersten elektrischen Parameter und dem Mischungsverhältnis der bekannten stofflichen Phasen ermittelbar ist. Das beschriebene Verfahren kommt beispielsweise bei magnetisch-induktiven Durchflussmessgeräten zum Einsatz, die prinzipbedingt Elektroden aufweisen, die in erster Linie zur Aufnahme induzierter elektrischer Spannungen in dem Medium dienen. Im Falle der Realisierung des Verfahrens zur Bestimmung des Volumenanteils einer Phase werden diese Elektroden verwendet, um das Medium mit dem elektrischen Anregungssignal zu beaufschlagen.

Problematisch ist bei dem beschriebenen Verfahren, dass die Zuverlässigkeit des Ergebnisses der Ermittlung der Volumenanteile der beteiligten Phasen stark davon abhängt, dass tatsächlich auch nur die angenommenen Phasen mit den wohlbekannten Werten für den ersten elektrischen Parameter der Phasen beteiligt sind und die am Mehrphasenmedium beteiligten Phasen unverändert bleiben. Dass sich zumindest eine der beteiligten Phasen in ihren Eigenschaften tatsächlich ändert (und damit auch in dem ersten elektrischen Parameter), kommt durchaus häufiger vor, als Beispiel sei hier ein Zweiphasenmedium aus Öl und Meerwasser genannt, wobei sich der Salzgehalt (und damit die elektrische Leitfähigkeit) des Meerwassers ändert. Die Bestimmung der Volumenanteile der Phasen des Mediums ist bei dem geschilderten Verfahren also sehr empfindlich gegenüber Änderungen der beteiligten Medien.

Aufgabe der vorliegenden Erfindung ist es daher, das Verfahren und auch das Messgerät zur Bestimmung des Volumenanteils einer Phase eines mehrphasigen Mediums so weiterzubilden, dass es weniger empfindlich ist gegenüber einer Änderung der beteiligten Phasen und ihrer Werte für den ersten elektrischen Parameter.

Die zuvor hergeleitete Aufgabe ist bei dem eingangs beschriebenen Verfahren zur Bestimmung des Volumenanteils einer Phase eines mehrphasigen Mediums und bei dem eingangs beschriebenen Messgerät gelöst mit den Merkmalen der Kennzeichnungsteile der unabhängigen Patentansprüche. Das erfindungsgemäße Verfahren zeichnet sich dadurch aus, dass aus der Mehrzahl an ermittelten Werten für den ersten elektrischen Parameter des Mediums ein Streuungswert eines statistischen Streuungsmaßes ermittelt wird und dass unter Verwendung eines mathematischen Zusammenhangs zwischen dem statistischen Streuungsmaß des ersten elektrischen Parameters und einem Volumenanteil der zweiten Phase in dem Medium mit dem ermittelten Streuungswert des ersten elektrischen Parameters der Volumenanteil der zweiten Phase in dem Medium bestimmt wird. Bemerkenswert an dem Verfahren ist, dass es losgelöst ist von den unmittelbaren Werten des ersten elektrischen Parameters (wie beispielsweise der ermittelten elektrischen Leitfähigkeit des Mediums oder der Impedanz des Mediums) und dass stattdessen ein statistisches Streuungsmaß des ersten elektrischen Parameters verwendet wird auf Grundlage einer Mehrzahl von Werten für den ersten elektrischen Parameter. Erfindungsgemäß ist erkannt worden, dass es einen Zusammenhang zwischen Streuungswerten dieses statistischen Streuungsmaßes und dem Volumenanteil der zweiten Phase in dem Medium gibt. Die Verwendung eines solchen Zusammenhangs an sich reduziert schon die Abhängigkeit von den absoluten Werten des ersten elektrischen Parameters des Mediums.

Gemäß einer vorteilhaften Ausgestaltung des Verfahrens ist das Anregungssignal eine elektrische Spannung oder ein elektrischer Strom. Entsprechend ist das elektrische Reaktionssignal ein elektrischer Strom oder eine elektrische Spannung, insbesondere wobei das Anregungssignal eine harmonische Schwingung mit einer Anregungsfrequenz ist. Die Wahl des Anregungssignals als eine harmonische Schwingung ermöglicht die Auswertung von ersten elektrischen Parametern, die als harmonische Wechselgrößen definiert sind.

Gemäß einer weiteren vorteilhaften Ausgestaltung des Verfahrens ist vorgesehen, dass die Beaufschlagung des Mediums mit den elektrischen Anregungssignalen in einem Zeitbereich erfolgt, in dem keine andere Messwerterfassung im Medium durchgeführt wird. Durch diese Maßnahme wird verhindert, dass sich verschiedene Messwerterfassungen gegenseitig beeinflussen und damit insgesamt zu einem schlechteren Messwertergebnis führen. Wenn das Verfahren bei einem magnetisch-induktiven Durchflussmessgerät durchgeführt wird, das - wie zuvor ausgeführt - schon über entsprechende Elektroden in Wirkzusammenhang mit dem Medium verfügt, dann wird das Verfahren zur Bestimmung des Volumenanteils einer Phase durchgeführt, wenn keine magnetisch induktive Durchflussmessung durchgeführt wird. Insbesondere wird das Verfahren dann durchgeführt nach einem Umschalten der Polarität des für die magnetisch-induktive Durchflussmessung stets erforderlichen Magnetfeldes, insbesondere im Zeitbereich eines nicht-stationären Magnetfeldes, da sich diese Zeitbereiche für eine Durchflussmessung wenig eignen.

Bei einer weiteren bevorzugten Ausgestaltung des Verfahrens wird als statistisches Streuungsmaß die Standardabweichung berechnet. Die Berechnung der Standardabweichung ist einfach, und es hat sich herausgestellt, dass die Standardabweichung in vielen Fällen eine gute Korrelation mit dem Volumenanteil der zweiten Phase des Mediums aufzeigt.

Ein Merkmal des erfindungsgemäßen Verfahrens ist, dass der erste elektrische Parameter des Mediums die elektrische Leitfähigkeit des Mediums und/oder der Betrag der Impedanz des Mediums und/oder der Phasenwinkel der Impedanz des Mediums ist, insbesondere wobei die elektrische Leitfähigkeit aus dem Realteil der Impedanz des Mediums unter Berücksichtigung der Geometrie des Messvolumens, in dem sich das Medium befindet, bestimmt wird.

Bei einer bevorzugten Ausgestaltung des Verfahrens lässt sich eine maßgebliche, noch weitergehende Unempfindlichkeit gegenüber einer Änderung der stofflichen Eigenschaften der beteiligten Phasen und ihrer Werte für den ersten elektrischen Parameter der Phasen realisieren, indem der ermittelte Streuungswert des ersten elektrischen Parameters normiert wird auf den Absolutwert des ersten elektrischen Parameters, insbesondere auf einen aus der Mehrzahl an ermittelten Werten für den ersten elektrischen Parameter des Mediums berechneten absoluten Mittelwert des ersten elektrischen Parameters. Durch die Normierung wird die Standardabweichung ebenfalls unabhängig von den Absolutwerten und möglichen Schwankungen in den Absolutwerten des ersten elektrischen Parameters der Phasen. Selbst wenn sich die stoffliche Zusammensetzung einer oder auch beider der beteiligten Phasen ändert, gibt es gleichwohl bei der Verwendung normierter Größen eine gute Korrelation zwischen der Änderung des ermittelten Streuungswerts des ersten elektrischen Parameters und dem Volumenanteil der zweiten Phase des Mediums.

Eine weitere bevorzugte Weiterbildung des Verfahrens ist dadurch gekennzeichnet, dass aus der Mehrzahl an Anregungssignalen und der Mehrzahl an Reaktionssignalen eine Mehrzahl von Werten für einen zweiten elektrischen Parameter des Mediums ermittelt wird, dass aus der Mehrzahl an ermittelten Werten für den zweiten elektrischen Parameter des Mediums ein Streuungswert eines statistischen Streuungsmaßes ermittelt wird, und dass unter Verwendung eines mathematischen Zusammenhangs zwischen dem statistischen Streuungsmaß des zweiten elektrischen Parameters und dem Volumenanteil der zweiten Phase in dem Medium mit dem ermittelten Streuungswert des zweiten elektrischen Parameters der Volumenanteil der zweiten Phase in dem Medium bestimmt wird. Der Zusammenhang zu dem Volumenanteil der zweiten Phase in dem Medium (und damit auch zu dem Volumenanteil der ersten Phase in dem Medium) zu dem Wert des verwendeten statistischen Streuungsmaßes von verschiedenen elektrischen Parametern des Mediums hat sich als weitreichend einsetzbar hinsichtlich verschiedener elektrischer Parameter herausgestellt, sodass auch weitere elektrische Parameter zu der Bestimmung des Volumenanteils der zweiten Phase des Mediums ohne Weiteres heranziehbar sind. Daraus ergeben sich vielfältige Möglichkeiten der gegenseitigen Überprüfung von Berechnungsergebnissen, also auch hinsichtlich der Redundanz und/oder Kombination von verschiedenen Berechnungsergebnissen auf verschiedenen Wegen und auch Möglichkeiten hinsichtlich der gegenseitigen Überprüfbarkeit und Plausibilitätskontrollen von auf verschiedenen Wegen gewonnenen Ergebnissen bezüglich des Volumenanteils der zweiten Phase des Mediums.

Die zuvor genannte bevorzugte Ausgestaltung des Verfahrens bietet die Möglichkeit hinsichtlich einer Weiterentwicklung des Verfahrens, dass aus dem mit dem ersten elektrischen Parameter des Mediums ermittelten Volumenanteil der zweiten Phase des Mediums und aus dem mit dem zweiten elektrischen Parameter des Mediums ermittelten Volumenanteil der zweiten Phase des Mediums ein gemittelter Volumenanteil der zweiten Phase des Mediums berechnet wird. Die Möglichkeit der Kombination auf Grundlage verschiedener elektrischer Parameter des Mediums ermittelter Volumenanteile der zweiten Phase des Mediums bietet sich deshalb an, weil sich der Zusammenhang zwischen dem Wert des verwendeten statistischen Streuungsmaßes eines elektrischen Parameters des Mediums und des Volumenanteils der zweiten Phase des Mediums als relativ universell herausgestellt hat.

Bei einer weiteren vorteilhaften Ausgestaltung des Verfahrens wird der mit dem ersten elektrischen Parameter des Mediums ermittelte Volumenanteil der zweiten Phase in dem Medium und/oder der mit dem zweiten elektrischen Parameter des Mediums ermittelte Volumenanteil der zweiten Phase des Mediums und/oder der gemittelte Volumenanteil der zweiten Phase in dem Medium signalisiert, insbesondere wird der ermittelte Volumenanteil in einem Speicher einer Steuer- und Auswerteeinheit des Messgeräts abgelegt wird und/oder angezeigt und/oder über eine Kommunikationsschnittstelle des Messgeräts eines Durchflussmessgeräts, insbesondere eines magnetisch-induktiven Durchflussmessgeräts, an einen angeschlossenen Kommunikationspartner übermittelt.

Bei einer weiteren bevorzugten Ausgestaltung des Verfahrens ist vorgesehen, dass bei Überschreiten eines ersten Grenzwertes durch den mit dem ersten elektrischen Parameter des Mediums ermittelten Volumenanteil der zweiten Phase in dem Medium und/oder bei Überschreiten eines zweiten Grenzwertes durch den mit dem zweiten elektrischen Parameter des Mediums ermittelten Volumenanteil der zweiten Phase des Mediums und/oder bei Überschreiten eines dritten Grenzwertes durch den gemittelten Volumenanteil der zweiten Phase in dem Medium das Vorliegen einer Zwei-Phasen-Strömung signalisiert wird, insbesondere in einem Speicher der Steuer- und Auswerteeinheit des Messgeräts abgelegt wird und/oder angezeigt wird und/oder über eine Kommunikationsschnittstelle des Messgeräts, insbesondere eines magnetisch-induktiven Durchflussmessgeräts, an einen angeschlossenen Kommunikationspartner übermittelt wird.

Bei einer bevorzugten Ausgestaltung des Verfahrens ist vorgesehen, dass der mathematische Zusammenhang zwischen dem statistischen Streuungsmaß des ersten elektrischen Parameters und dem Volumenanteil der zweiten Phase des Mediums und/oder der mathematische Zusammenhang zwischen dem statistischen Streuungsmaß des zweiten elektrischen Parameters und dem Volumenanteil der zweiten Phase des Mediums ermittelt wird, indem der Streuungswert des ersten elektrischen Parameters und/oder der Streuungswert des zweiten elektrischen Parameters bei zumindest zwei verschiedenen aber bekannten Volumenanteilen der zweiten Phase ermittelt werden, insbesondere wobei eine lineare Abhängigkeit zwischen dem statistischen Streuungsmaß des ersten elektrischen Parameters und dem Volumenanteil der zweiten Phase des Mediums und/oder zwischen dem statistischen Streuungsmaß des zweiten elektrischen Parameters und dem Volumenanteil der zweiten Phase des Mediums angenommen wird. Wenn es lediglich zwei Stützstellen gibt, dann kann daraus als Zusammenhang zwischen dem Volumenanteil der zweiten Phase des Mediums und dem Streuungswert des statistischen Streuungsma-βes des zweiten Mediums sofort eine Geradengleichung abgeleitet werden. Bei mehr als zwei Stützstellen kann der Zusammenhang linear als Regressionsgerade formuliert werden. Es hat sich gezeigt, dass die Annahme derartig linearer Zusammenhänge eine geeignete Annäherung des tatsächlichen Verhaltens darstellt.

In einer weiteren bevorzugten Ausgestaltung des Verfahrens werden mehr als zwei Stützstellen aufgenommen, also mehr als zwei Zusammenhänge zwischen dem statistischen Streuungsmaß eines elektrischen Parameters und dem Volumenanteil der zweiten Phase des Mediums, und es wird als mathematischer Zusammenhang zwischen dem statistischen Streuungsmaß eines elektrischen Parameters und dem Volumenanteil der zweiten Phase des Mediums eine polynomiale Beschreibung des Zusammenhangs ermittelt oder eine Beschreibung des Zusammenhangs wird anhand einer Spline-Interpolationen ermittelt, insbesondere unter Optimierung der Beschreibungen der Zusammenhänge anhand der Minimierung eines Abweichungsmaßes.

Bei dem eingangs beschriebenen Messgerät wird die hergeleitete Aufgabe dadurch gelöst, dass die Steuer- und Auswerteeinheit so ausgestaltet ist, dass das Messgerät im Betrieb das zuvor ausführlich beschriebene Verfahren durchführt. Insbesondere wird das Verfahren durchgeführt bei einem Durchflussmessgerät, insbesondere bei einem magnetisch-induktiven Durchflussmessgeräte, bei dem die erforderlichen Elektroden ohnehin schon vorgesehen sind, oder bei einem Coriolis-Massedurchflussmessgerät, einem Vortex-Durchflussmessgerät, einem Magnet-Resonanz-Durchflussmessgerät oder einem Schwebekörper-Durchflussmessgerät. Bei den letztgenannten Durchflussmessgeräten sind die für die Durchführung des Verfahrens erforderlichen Elektroden nicht zwingend implementiert, gegebenenfalls müssen sie zusätzlich vorgesehen werden.

Im Einzelnen gibt es nun eine Vielzahl von Möglichkeiten, das erfindungsgemäße Verfahren und das erfindungsgemäße Messgerät auszugestalten und weiterzubilden. Dazu wird verwiesen einerseits auf die den unabhängigen Patentansprüchen nachgeordneten Patentansprüche, andererseits auf die folgende Beschreibung von Ausführungsbeispielen in Verbindung mit der Zeichnung. In der Zeichnung zeigen
- Fig. 1: schematisch in Teilaspekten die Grundlagen eines Verfahrens zur Bestimmung des Volumenanteils einer Phase eines mehrphasigen Mediums,
- Fig. 2: schematisch die Schritte eines Verfahrens zur Bestimmung des Volumenanteils einer Phase eines mehrphasigen Mediums,
- Fig. 3: schematisch das Verfahren zur Bestimmung des Volumenanteils einer Phase eines mehrphasigen Mediums anhand der elektrischen Leitfähigkeit,
- Fig. 4: schematisch das Verfahren zur Bestimmung des Volumenanteils einer Phase eines mehrphasigen Mediums anhand der elektrischen Impedanz,
- Fig. 5: schematisch das Verfahren zur Bestimmung des Volumenanteils einer Phase eines mehrphasigen Mediums anhand des Absolutwertes der elektrischen Impedanz,
- Fig. 6: schematisch das Verfahren zur Bestimmung des Volumenanteils einer Phase eines mehrphasigen Mediums anhand der Phaseninformation der elektrischen Impedanz und
- Fig. 7: schematisch ein Messgerät zur Bestimmung des Volumenanteils einer Phase eines mehrphasigen Mediums anhand der zuvor beschriebenen Verfahren.

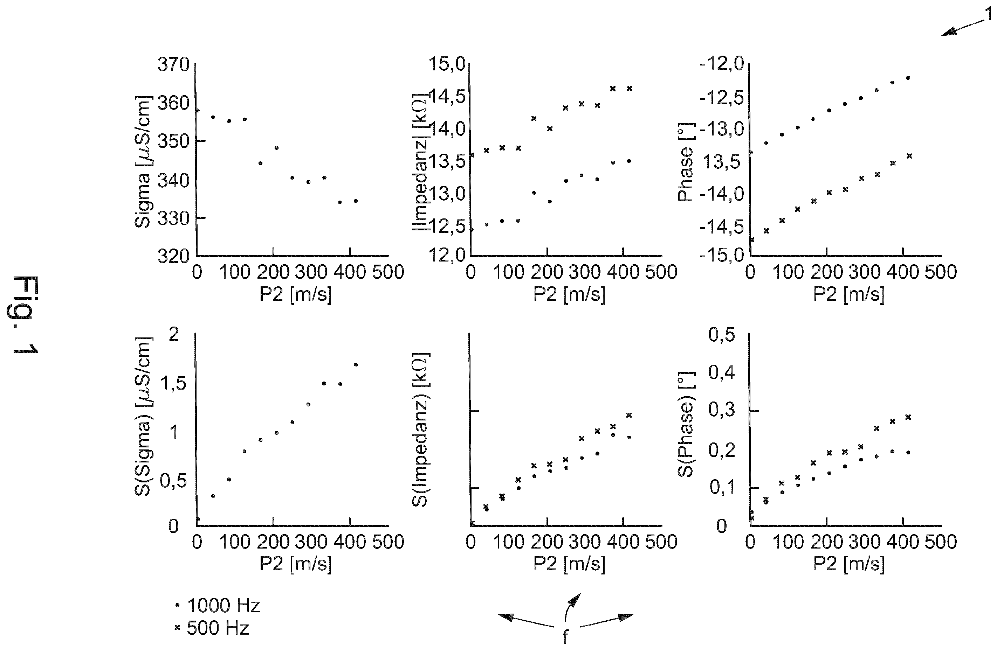

Die Fig. 1 bis 7 zeigen in unterschiedlichen Aspekten ein Verfahren 1 und ein Messgerät 2 zur Bestimmung des Volumenanteils V% einer Phase eines mehrphasigen Mediums M, wobei das Medium M eine erste Phase P1 mit einem ersten Wert E1 für einen ersten elektrischen Parameter E und zumindest zeitweilig eine zweite Phase P2 mit einem zweiten Wert E2 für den ersten elektrischen Parameter E aufweist.

In den Figuren werden als Bezugszeichen sowohl Zahlen als auch Buchstaben verwendet. Die Buchstaben haben größtenteils nur den Charakter von Bezugszeichen, sie erleichtern das Verständnis und die Herstellung des Zusammenhangs von Beschreibung und Zeichnung aber ganz erheblich. Teilweise haben die Bezugszeichen zusätzlich auch den Charakter von Formelzeichen, wobei die Formelzeichen beispielsweise in den Ansprüchen hilfreich sind zum Verständnis der Ansprüche, sie sind aber nicht notwendig für das Verständnis. Insoweit sind die Buchstaben-Bezugszeichen wie auch Formelzeichen in den Ansprüchen in Klammern gesetzt und werden auch in der nachfolgenden Figurenbeschreibung wie Bezugszeichen behandelt.

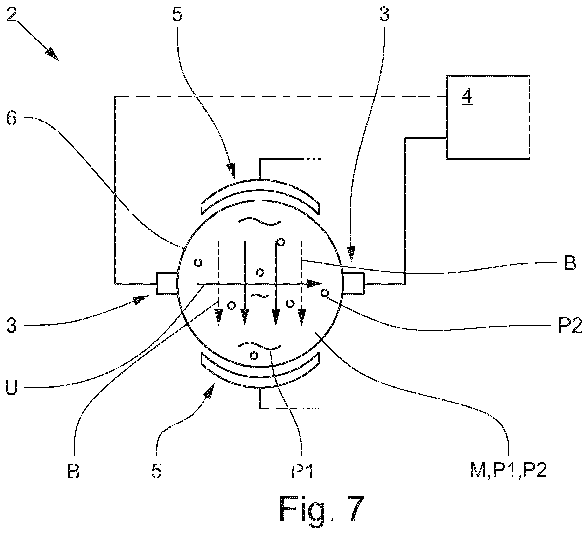

Es handelt sich bei den betrachteten Messungen um häufig anzutreffende Messaufgaben, bei denen der Anteil der verschiedenen Phasen P1, P2 an dem Medium M von Interesse ist. Wenn beispielsweise gleichzeitig ein Volumenstrom gemessen wird, kann die Information über die Phasenanteile der Phasen P1, P2 an dem Medium M für den Messvorgang entscheidend sein, beispielsweise zu Zwecken einer Abrechnung über gelieferte Rohstoffe; dies leuchtet unmittelbar ein, wenn es sich zum Beispiel bei der ersten Phase P1 des Mediums M um flüssiges Rohöl und bei der zweiten Phase P2 des Mediums M um Wasser handelt (Fig. 7).

Während die Fig. 1 bis 6 eher verfahrensmäßige Merkmale verdeutlichen, ist in Fig. 7 ein Messgerät 2 zur Bestimmung des Volumenanteils V% einer Phase des mehrphasigen Mediums M gezeigt.

Zur Bestimmung des Volumenanteils V% einer Phase des mehrphasigen Mediums M ist es bekannt, das Medium M über ein Elektrodenpaar 3 mit einer Mehrzahl elektrischer Anregungssignale zu beaufschlagen und zu den elektrischen Anregungssignalen eine Mehrzahl entsprechender elektrischer Reaktionssignale zu erfassen. Aus der Mehrzahl an Anregungssignalen und der Mehrzahl an Reaktionssignalen wird eine Mehrzahl von Werten En für den ersten elektrischen Parameter E des Mediums M ermittelt. Wenn es sich um bekannte Phasenbestandteile P1, P2 mit bekannten Werten für den ersten elektrischen Parameter E, beispielsweise die spezifische elektrische Leitfähigkeiten, für die Phasen P1, P2 in Reinform handelt, dann leuchtet es ein, dass ein sich verändernder Anteil der Phasen P1, P2 an dem Medium M auch zu einem sich verändernden Wert für den ersten elektrischen Parameter E des Mediums M führt, und eine Bestimmung des ersten elektrischen Parameters E des Mediums M auch eine Bestimmung beispielsweise des Volumenanteils V%_P2 der zweiten Phase P2 des Mediums M ermöglicht.

Bei dem Messgerät 2 in Fig. 7 handelt es sich um ein magnetisch-induktives Durchflussmessgerät mit einem zur Aufnahme eines Mediums M dienenden Messvolumen 6, das vorliegend ein von dem Medium M durchströmtes Messrohr ist. Das Medium M weist eine erste Phase P1 mit einem ersten Wert E1 für einen ersten elektrischen Parameter E und zumindest zeitweilig eine zweite Phase P2 mit einem zweiten Wert E2 für den ersten elektrischen Parameter E auf. Das Messgerät 2 weist eine Steuer- und Auswerteeinheit 4 und ein mit dem Medium M in Kontakt stehendes Elektrodenpaar 3 auf, wobei die Steuer- und Auswerteeinheit 4 im Betriebszustand des Messgeräts 2 zur Erfüllung der primären Messaufgabe, nämlich der Messung eines Volumendurchflusses durch das Messrohr 6, mittels einer Magnetfelderzeugungsvorrichtung 5 ein Magnetfeld B erzeugt, durch das - ein leitfähiges Medium M vorausgesetzt - eine von der Fließgeschwindigkeit abhängige und zu der Fließgeschwindigkeit proportionale Messspannung U in dem Medium M erzeugt wird. Diese Messspannung U wird über das Elektrodenpaar 3 abgegriffen. So wird eine Durchflussinformation mithilfe des Messgeräts 2, das in dem Ausführungsbeispiel ein magnetisch-induktives Durchflussmessgerät ist, gewonnen. Das magnetisch-induktive Durchflussmessgerät ist ein typisches Beispiel für ein Messgerät 2, das bereits über ein Elektrodenpaar 3 verfügt, wenngleich dieses Elektrodenpaar 3 bei der originären Messaufgabe - der Durchflussmessung - eine andere Aufgabe hat, nämlich die passive Aufnahme der induzierten Messspannung U in dem Medium M.

Messgeräte 2 müssen nicht wie bei dem in Fig. 7 dargestellten Beispiel eine zusätzliche Messaufgabe wahrnehmen, sie können auch einfach dazu dienen, den Volumenanteil V% einer Phase des mehrphasigen Mediums M zu bestimmen.

Zur Bestimmung des Volumenanteils V% einer Phase des mehrphasigen Mediums M wird das Medium M über das Elektrodenpaar 3 mit einer Mehrzahl elektrischer Anregungssignale beaufschlagt und zu den elektrischen Anregungssignalen wird eine Mehrzahl entsprechender elektrischer Reaktionssignale erfasst. Aus der Mehrzahl an Anregungssignalen und der Mehrzahl an Reaktionssignalen wird eine Mehrzahl von Werten En für den ersten elektrischen Parameter E des Mediums M ermittelt. Im Stand der Technik wird als erster elektrischer Parameter E beispielsweise die Leitfähigkeit des Mediums M bestimmt und aus der ermittelten Leitfähigkeit des Mediums wird auf den Volumenanteil V% der Beteiligten Phasen P1, P2 des Mediums M geschlossen.

Die direkte Bestimmung des Volumenanteils V% einer Phase des Mediums M hat verschiedene Nachteile, die teilweise mit einer stofflichen Änderung zumindest einer der beteiligten Phasen des Mediums M in dem betrachteten Messvolumen 6 in Zusammenhang stehen und damit mit einer Änderung der Eigenschaften des ersten elektrischen Parameters E der beteiligten Phasen P1, P2.

Das hier beschriebene Verfahren 1 geht einen deutlich anderen Weg. Das Verfahren 1 sieht vor, dass aus der Mehrzahl an ermittelten Werten En für den ersten elektrischen Parameter E des Mediums M ein Streuungswert S(En) eines statistischen Streuungsmaßes S ermittelt wird, und dass unter Verwendung eines mathematischen Zusammenhangs f zwischen dem statistischen Streuungsmaß S des ersten elektrischen Parameters E und einem Volumenanteil V%_P2 der zweiten Phase in dem Medium M mit dem ermittelten Streuungswert S(En) des ersten elektrischen Parameters der Volumenanteil V%_P2 der zweiten Phase P2 in dem Medium M bestimmt wird.

Die dem Verfahren 1 zugrunde liegende Erkenntnis besteht darin, dass nicht nur der unmittelbare Wert - wie aus dem Stand der Technik bekannt - des ersten elektrischen Parameters E des Mediums M Auskunft über den Volumenanteil V%_P2 der zweiten Phase P2 in dem Medium gibt, sondern überraschenderweise auch der Streuungswert S(En) des statistischen Streuungsmaßes S, beruhend auf einer Mehrzahl von Messwerten des ersten elektrischen Parameters E. Damit wird die Bestimmung des Volumenanteils V%_P2 der zweiten Phase P2 in dem Medium M zu einem gewissen Grad unabhängig von den absoluten Werten für den ersten elektrischen Parameter E des Mediums M.

Die dem Verfahren 1 zugrunde liegenden aufgefundenen Zusammenhänge sind in Fig. 1 beispielhaft an realen Messungen mit einem magnetisch-induktiven Durchflussmessgerät 2, wie in Fig. 7 gezeigt, dargestellt. Es sind verschiedene Messungen für den ersten elektrischen Parameter E des Mediums M durchgeführt worden. Zum einen ist als erster elektrischer Parameter E die Leitfähigkeit sigma des Mediums M bestimmt worden und zum anderen die Impedanz des Mediums, die eine Wechselstromgröße ist und als solche einen Betrag (abs(Impedanz)) und eine Phase aufweist. Diese Messungen sind in den Diagrammen der oberen Zeile von Fig. 1 dargestellt. In der unteren Zeile von Fig. 1 sind die Standardabweichungen S einer Mehrzahl von Messungen En des entsprechenden ersten elektrischen Parameters E in Form der elektrischen Leitfähigkeit sigma und des Absolutwertes und der Phase der Impedanz des Mediums M dargestellt. Die Messungen sind bei verschiedenen Frequenzen durchgeführt worden.

Die Messungen in Fig. 1 sind so durchgeführt worden, dass bei einem gleichbleibenden Volumenstrom der ersten leitfähigen Phase P1 des Mediums (Wasser, flüssig bei 2,4 l/s) stets die Beimischung der zweiten Phase P2 von Luft (gasförmig) erhöht worden ist. Insoweit entspricht die Veränderung des Volumenstroms einer Phase sinngemäß auch einer Veränderung des Volumenanteils dieser Phase. Dargestellt sind sowohl Messungen der Leitfähigkeit sigma des Mediums M wie auch die der komplexwertigen Impedanz des Mediums, hier aufgeteilt in den Absolutwert der Impedanz und die Phase der Impedanz. Für jede Beimischung der zweiten Phase (Luft) P2 des Mediums M ist eine Mehrzahl von Werten En der genannten elektrischen Parameter E des Mediums M aufgenommen worden. Aus der Mehrzahl an ermittelten Werten En für den ersten elektrischen Parameter E des Mediums M ist dann ein Streuungswert S(En) des statistischen Streuungsmaßes S ermittelt worden. Vorliegend ist als statistisches Streuungsmaß S die Standardabweichung gewählt worden. Dass ein - in vielen Fällen oft linear annäherbarer - Zusammenhang f besteht zwischen dem Volumenanteil V%_P2 der zweiten Phase in dem Medium M (der unmittelbar hervorgeht aus dem beigemischt Anteil der Luft der zweiten Phase P2) und der Standardabweichung S der entsprechenden Werte S(En) für den ersten elektrischen Parameter E ist überraschend aber gleichwohl erkennbar.

Bei den hier durchgeführten Verfahren 1 ist das Anregungssignal eine elektrische Spannung oder ein elektrischer Strom und das elektrische Reaktionssignal ist demzufolge ein elektrischer Strom oder eine elektrische Spannung. Wenn die elektrischen Parameter E des Mediums M Wechselstromgrößen sind, wie beispielsweise die Impedanz oder die Admittanz, dann ist das Anregungssignal üblicherweise eine harmonische Schwingung mit einer dazugehörigen Anregungsfrequenz.

Die Beaufschlagung des Mediums M mit den elektrischen Anregungssignalen erfolgt in einem Zeitbereich, in dem keine andere Messwerterfassung im Medium M durchgeführt wird, wobei speziell bei dem magnetisch-induktiven Durchflussmessgerät 2 gemäß Fig. 7 die Messungen durchgeführt werden nach einem Umschalten der Polarität des Magnetfeldes B, das durch die Magnetfeldeinrichtung 5 erzeugt wird, nämlich im Zeitbereich eines nicht-stationären Magnetfeldes B. Wird zur Durchführung des Verfahrens 1 ein Messgerät verwendet, das überhaupt keine andere Messaufgabe hat, das also nur zur Bestimmung des Volumenanteils V% einer Phase eines mehrphasigen Mediums M dient, oder lediglich eine Messaufgabe hat, die physikalisch nicht mit der Bestimmung des Volumenanteils kollidiert, dann muss selbstverständlich nicht auf eine mögliche Kollision mit einer anderen Messwerterfassung geachtet werden.

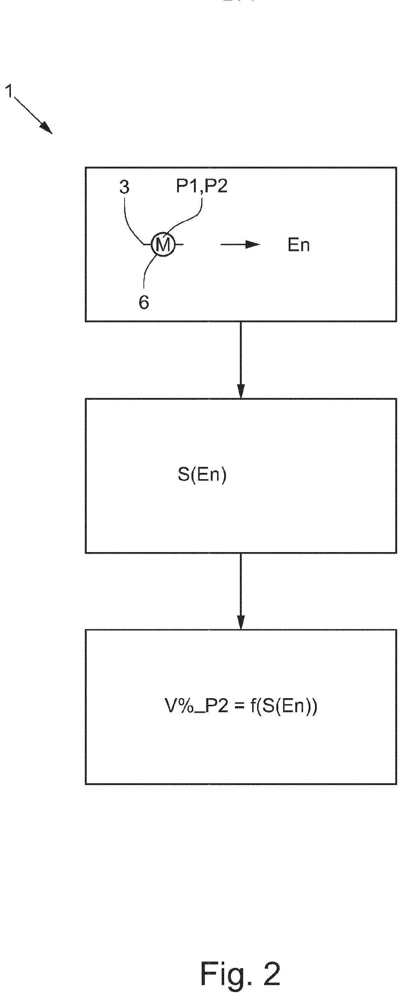

Fig. 2 zeigt die wesentlichen Verfahrensschritte zur Durchführung des Verfahrens 1. Im obersten Verfahrensschritt ist dargestellt, dass sich das mehrphasigen Medium M mit den Phasen P1 und P2 in einem Messvolumen 6 befindet. Mit einem Elektrodenpaar 3 wird das Medium M mit einer Mehrzahl elektrischer Anregungssignale beaufschlagt und zu den elektrischen Anregungssignalen wird eine Mehrzahl entsprechender elektrischer Reaktionssignale erfasst. Aus den Signalen wird eine Mehrzahl von Werten En für den ersten elektrischen Parameter jedes Mediums ermittelt, beispielsweise die elektrische Leitfähigkeit, die Impedanz, die Admittanz usw. In dem mittleren Verfahrensschritt wird ein Streuungswert S(En) des statistischen Streuungsmaßes S, beispielsweise die Standardabweichung, aus der Mehrzahl von Werten En des ersten elektrischen Parameters E ermittelt. Im letzten Verfahrensschritt wird schließlich unter Verwendung des mathematischen Zusammenhangs f zwischen dem statistischen Streuungsmaß S bzw. dem daraus ermittelten Streuungswert S(En) und dem Volumenanteil V%_P2 der zweiten Phase in dem Medium M der konkrete Volumenanteil V%_P2(S(En)) der zweiten Phase P2 ermittelt.

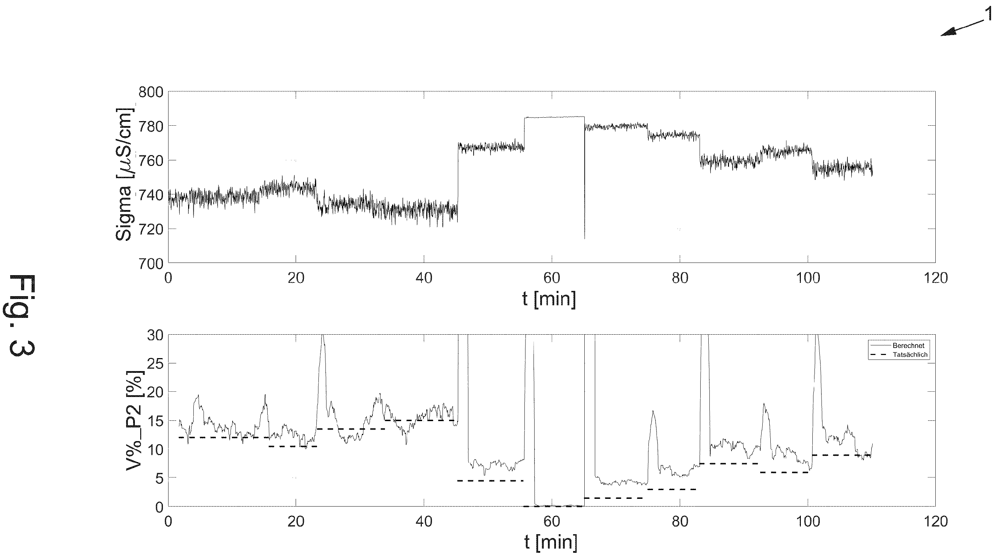

Die Fig. 3 bis 6 zeigen mit dem Verfahren 1 gewonnene Messergebnisse hinsichtlich des Volumenanteils V%_P2 der zweiten Phase P2 des Mediums M. In dem jeweils oberen Diagramm der Figuren sind die gemessenen Werte der ersten elektrischen Größe dargestellt über der Zeit t über einen relativ großen Zeitraum von fast zwei Stunden hinweg. Das jeweils untere Diagramm zeigt den korrespondierenden Verlauf des tatsächlichen (gestrichelte Linie) und des ermittelten Volumenanteils V%_P2 der zweiten Phase P2 des Mediums M. Der tatsächliche Verlauf des Volumenanteils V%_P2 des Mediums M ist über weite Zeitbereiche hinweg konstant. Die Messungen sind in einer Messeanordnung entstanden, die es gestattet, die Volumenanteile V% der Phasen P1, P2 des Mediums M genau vorzugeben. Als Messgerät 2 ist ein magnetisch-induktives Messgerät gemäß Fig. 7 zum Einsatz gekommen, dessen Steuer-und Auswerteeinheit 4 so ausgestaltet ist, dass es im Betrieb das zuvor beschriebene Verfahren 1 ausführen kann.

Bei dem Verfahren 1 gemäß Fig. 3 ist als erster elektrischer Parameter E des Mediums M die elektrische Leitfähigkeit sigma des Mediums M verwendet worden.

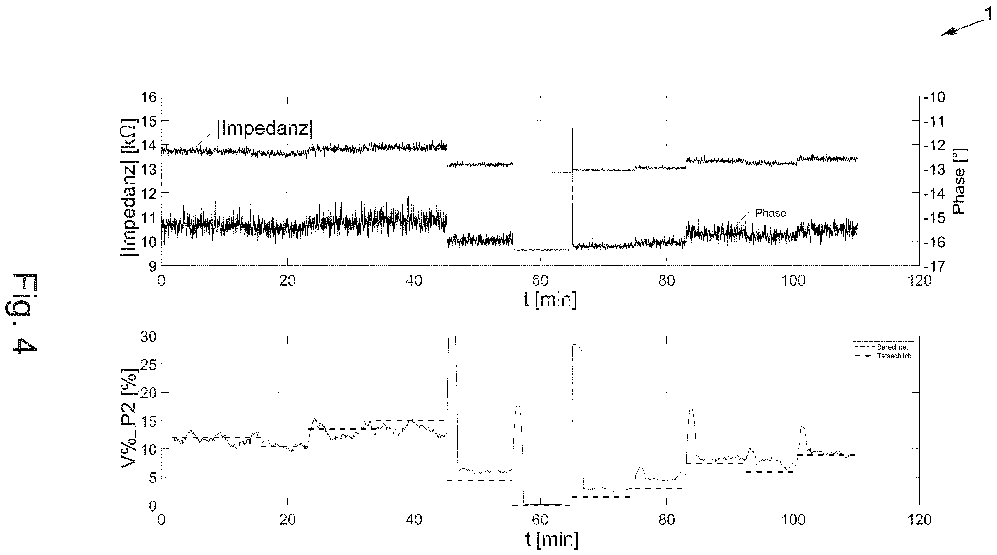

Bei dem Verfahren 1 gemäß Fig. 4 ist als erster elektrischer Parameter E des Mediums M die Impedanz des Mediums M verwendet worden, also ein zweiwertiger elektrischer Parameter, der in Zusammenhang mit harmonischen Wechselgrößen als Anregungs- und Reaktionssignale definiert ist. Insoweit sind in der oberen Darstellung von Fig. 4 der Betrag der Impedanz und die Phase der Impedanz dargestellt.

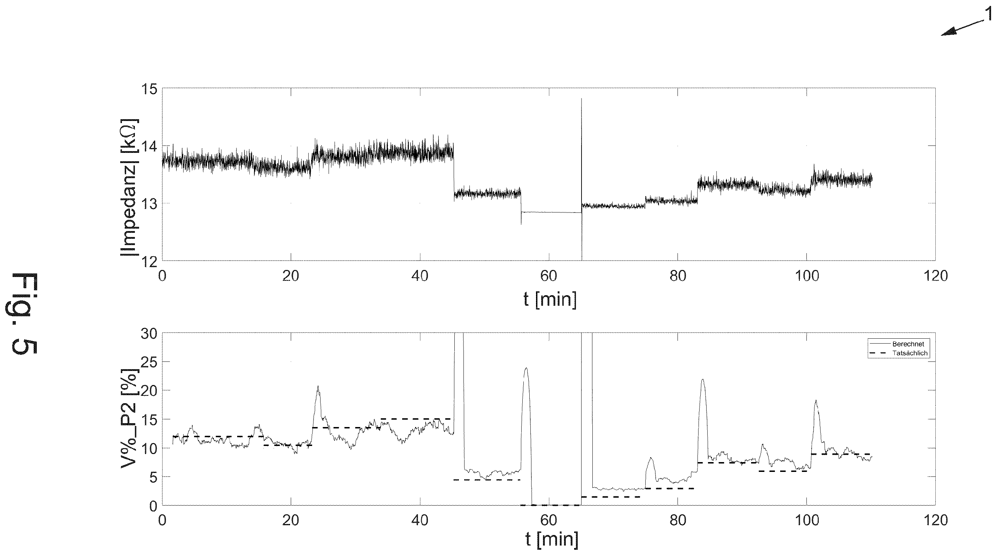

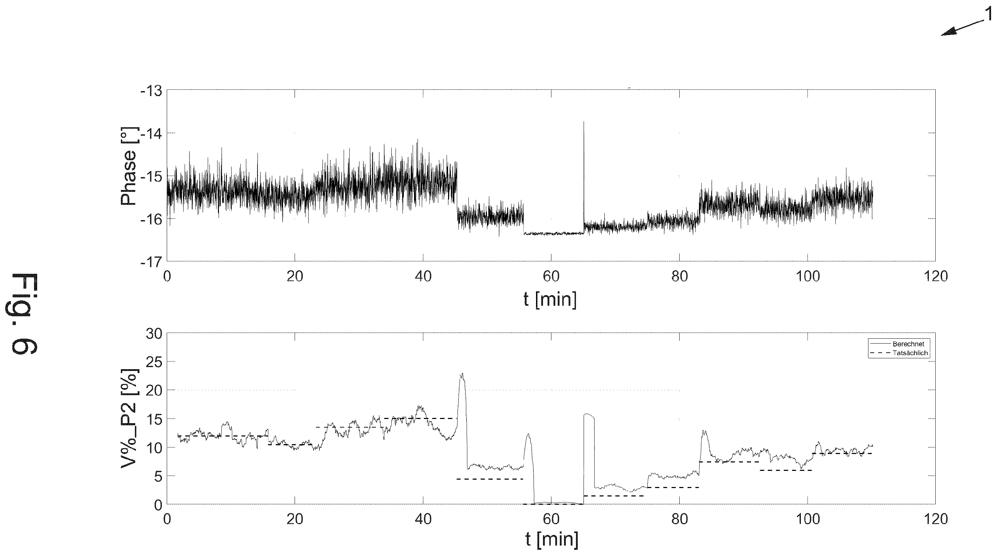

In den Fig. 5 und 6 wird nur mit einer der beiden Größen der komplexwertigen Impedanz gearbeitet, in Fig. 5 mit dem Betrag der Impedanz und in Fig. 6 mit der Phase der Impedanz.

Anhand der Darstellungen ist jedenfalls erkennbar, dass sich das Verfahren auf Grundlage der Verwendung eines Wertes S(En) eines statistischen Streuungsmaßes S auf Grundlage von ermittelten Werten En für den ersten elektrischen Parameter E sehr gut eignet, um den Volumenanteil V%_P2 der zweiten Phase P2 des mehrphasigen Mediums M auch quantitativ zu bestimmen.

Bei den Verfahren 1 gemäß den Fig. 3 und 5 ist ferner der ermittelte Streuungswert S(En) des ersten elektrischen Parameters E (elektrische Leitfähigkeit, Absolutwert der Impedanz) normiert worden auf den Absolutwert des ersten elektrischen Parameters E, vorliegend jeweils auf einen aus der Mehrzahl an ermittelten Werten En für den ersten elektrischen Parameter E des Mediums M berechneten absoluten Mittelwert des ersten elektrischen Parameters E. Dadurch wird eine erhebliche Unempfindlichkeit gegenüber stofflichen Veränderungen der Phasen P1, P2 des Mediums M erreicht, da es durch die Normierung auf die absoluten Werte des ersten elektrischen Parameters nicht mehr ankommt. Eine derartige Normierung des Phasenwinkels ist natürlich nicht erforderlich, da dieser unabhängig von den stofflichen Eigenschaften des Mediums immer nur in einem begrenzten absoluten Bereich liegen kann.

Bei einer bevorzugten, hier aber nicht ausdrücklich dargestellten Realisierung des Verfahrens 1 wird aus der Mehrzahl an Anregungssignalen und der Mehrzahl an Reaktionssignalen eine Mehrzahl von Werten für einen zweiten elektrischen Parameter des Mediums M ermittelt. Wenn als erster elektrischer Parameter beispielsweise der Betrag der Impedanz verwendet wird, könnte als zweiter elektrischer Parameter der Phasenwinkel der Impedanz verwendet werden. Aus der Mehrzahl an ermittelten Werten für den zweiten elektrischen Parameter des Mediums M wird dann ebenfalls ein Streuungswert eines statistischen Streuungsmaßes ermittelt. Unter Verwendung eines mathematischen Zusammenhangs zwischen dem statistischen Streuungsmaß des zweiten elektrischen Parameters und dem Volumenanteil der zweiten Phase in dem Medium M wird mit dem ermittelten Streuungswert des zweiten elektrischen Parameters der Volumenanteil der zweiten Phase in dem Medium bestimmt. Bei dieser Ausführung bietet es sich an, dass aus dem mit dem ersten elektrischen Parameter E des Mediums M ermittelte Volumenanteil V%_P2 der zweiten Phase P2 des Mediums M und aus dem mit dem zweiten elektrischen Parameter des Mediums ermittelte Volumenanteil der zweiten Phase des Mediums ein gemittelter Volumenanteil der zweiten Phase des Mediums zu berechnen.

Das in dem Messgerät 2 implementierte Verfahren 1 sieht vor, dass der mit dem ersten elektrischen Parameter E des Mediums M ermittelte Volumenanteil V%_P2 der zweiten Phase P2 in dem Medium M und/oder der mit dem zweiten elektrischen Parameter des Mediums ermittelte Volumenanteil der zweiten Phase des Mediums M und/oder der gemittelte Volumenanteil der zweiten Phase P2 in dem Medium M signalisiert wird, nämlich wahlweise in einem Speicher der Steuer- und Auswerteeinheit 4 abgelegt wird und/oder angezeigt wird und/oder über eine Kommunikationsschnittstelle des Messgeräts 2 an einen angeschlossenen Kommunikationspartner übermittelt wird.

Die Implementierung des Verfahrens 1 in dem Messgerät 2 sieht ferner vor, dass bei Überschreiten eines ersten Grenzwertes durch den mit dem ersten elektrischen Parameter E des Mediums M ermittelten Volumenanteil V%_P2 der zweiten Phase P2 in dem Medium M und/oder bei Überschreiten eines zweiten Grenzwertes durch den mit dem zweiten elektrischen Parameter des Mediums ermittelten Volumenanteil der zweiten Phase des Mediums M und/oder bei Überschreiten eines dritten Grenzwertes durch den gemittelten Volumenanteil der zweiten Phase in dem Medium das Vorliegen einer Zwei-Phasen-Strömung signalisiert wird, nämlich wahlweise in einem Speicher der Steuer- und Auswerteeinheit 4 abgelegt wird und/oder angezeigt wird und/oder über eine Kommunikationsschnittstelle des Messgeräts 2 an einen angeschlossenen Kommunikationspartner übermittelt wird.

Unter Berücksichtigung der Darstellung der funktionalen Zusammenhänge f in Fig. 1 (untere drei Abbildungen), ist nachvollziehbar, dass der mathematische Zusammenhang f zwischen dem statistischen Streuungsmaß S des ersten elektrischen Parameters E und dem Volumenanteil V%_P2 der zweiten Phase des Mediums M ermittelt worden ist, indem der Streuungswert S(En) des ersten elektrischen Parameters E bei zumindest zwei verschiedenen aber bekannten Volumenanteilen V%_P2 der zweiten Phase P2 des Mediums M ermittelt worden ist, wobei die Volumenanteile V% in Fig. 1 den Strömungsbestandteilen der zweiten Phase P2 des Mediums M entsprechen. In den Ausführungsbeispielen ist eine lineare Abhängigkeit zwischen dem statistischen Streuungsmaß S des ersten elektrischen Parameters E und dem Volumenanteil V%_P2 der zweiten Phase P2 des Mediums M angenommen worden.

### Bezugszeichen

- 1: Verfahren
- 2: Messgerät
- 3: Elektrodenpaar
- 4: Steuer- und Auswerteeinheit
- 5: Magnetfeldeinrichtung
- 6: Messvolumen, Messrohr

- B: Magnetfeld
- U: induzierte Spannung
- V%: Volumenanteil
- M: mehrphasiges Medium
- P1, P2: erste und zweite Phase des Mediums
- E: erster elektrischer Parameter des Mediums
- E1: erster Wert für den ersten elektrischen Parameter der ersten Phase
- E2: zweiter Wert für den ersten elektrischen Parameter der zweiten Phase
- En: Mehrzahl an ermittelten Werten für den ersten elektrischen Parameter
- S: statistisches Streuungsmaß
- S(En): Streuungswert für die Mehrzahl von ermittelten Werten En für den ersten elektrischen Parameter
- f: Zusammenhang zwischen dem statistischen Streuungsmaß S des ersten elektrischen Parameters E und dem Volumenanteil V%_P2 der zweiten Phase in dem Medium M
- V%_P2: Volumenanteil der zweiten Phase P2 in dem Medium M
- sigma: elektrische Leitfähigkeit

## Patentansprüche

1. Verfahren (1) zur Bestimmung des Volumenanteils (V%) einer Phase eines mehrphasigen Mediums (M), wobei das Medium (M) eine erste Phase (P1) mit einem ersten Wert (E1) für einen ersten elektrischen Parameter (E) und zumindest zeitweilig eine zweite Phase (P2) mit einem zweiten Wert (E2) für den ersten elektrischen Parameter aufweist, wobei das Medium (M) über ein Elektrodenpaar (3) mit einer Mehrzahl elektrischer Anregungssignale beaufschlagt wird und zu den elektrischen Anregungssignalen eine Mehrzahl entsprechender elektrischer Reaktionssignale erfasst wird, wobei aus der Mehrzahl an Anregungssignalen und der Mehrzahl an Reaktionssignalen eine Mehrzahl von Werten (En) für den ersten elektrischen Parameter (E) des Mediums (M) ermittelt wird,
wobei aus der Mehrzahl an ermittelten Werten (En) für den ersten elektrischen Parameter (E) des Mediums (M) ein Streuungswert (S(En)) eines statistischen Streuungsmaßes (S) ermittelt wird,
wobei unter Verwendung eines mathematischen Zusammenhangs (f) zwischen dem statistischen Streuungsmaß (S) des ersten elektrischen Parameters (E) und einem Volumenanteil (V%_P2) der zweiten Phase (P2) in dem Medium (M) mit dem ermittelten Streuungswert (S(En)) des ersten elektrischen Parameters (E) der Volumenanteil (V%_P2) der zweiten Phase (P2) in dem Medium (M) bestimmt wird und
wobei der erste elektrische Parameter (E) des Mediums (M) die elektrische Leitfähigkeit (sigma) des Mediums (M) und/oder der Betrag der Impedanz des Mediums (M) und/oder der Phasenwinkel der Impedanz des Mediums (M) ist.

2. Verfahren (1) nach Anspruch 1, **dadurch gekennzeichnet, dass** das Anregungssignal eine elektrische Spannung oder ein elektrischer Strom ist, und dass das elektrische Reaktionssignal ein elektrischer Strom oder eine elektrische Spannung ist, insbesondere wobei das Anregungssignal eine harmonische Schwingung mit einer Anregungsfrequenz ist.

3. Verfahren (1) nach Anspruch 1 oder 2, **dadurch gekennzeichnet, dass** die Beaufschlagung des Mediums (M) mit den elektrischen Anregungssignalen in einem Zeitbereich erfolgt, in dem keine andere Messwerterfassung im Medium (M) durchgeführt wird, dass insbesondere bei einem magnetisch-induktiven Messgerät keine magnetisch induktive Durchflussmessung durchgeführt wird, dann insbesondere nach einem Umschalten der Polarität des Magnetfeldes (B), insbesondere im Zeitbereich eines nicht-stationären Magnetfeldes (B).

4. Verfahren (1) nach einem der Ansprüche 1 bis 3, **dadurch gekennzeichnet, dass** als statistisches Streuungsmaß (S) die Standardabweichung berechnet wird.

5. Verfahren (1) nach einem der Ansprüche 1 bis 4, **dadurch gekennzeichnet, dass** die elektrische Leitfähigkeit aus dem Realteil der Impedanz des Mediums (M) unter Berücksichtigung des Durchmessers eines Messvolumens (6) für das Medium (M) bestimmt wird.

6. Verfahren (1) nach einem der Ansprüche 1 bis 5, **dadurch gekennzeichnet, dass** der ermittelte Streuungswert (S(En)) des ersten elektrischen Parameters normiert wird auf den Absolutwert des ersten elektrischen Parameters (E), insbesondere auf einen aus der Mehrzahl an ermittelten Werten für den ersten elektrischen Parameter (E) des Mediums (M) berechneten absoluten Mittelwert des ersten elektrischen Parameters (E).

7. Verfahren (1) nach einem der Ansprüche 1 bis 6, **dadurch gekennzeichnet, dass** aus der Mehrzahl an Anregungssignalen und der Mehrzahl an Reaktionssignalen eine Mehrzahl von Werten für einen zweiten elektrischen Parameter des Mediums (M) ermittelt wird, dass aus der Mehrzahl an ermittelten Werten für den zweiten elektrischen Parameter des Mediums (M) ein Streuungswert eines statistischen Streuungsmaßes ermittelt wird, und dass unter Verwendung eines mathematischen Zusammenhangs zwischen dem statistischen Streuungsmaß des zweiten elektrischen Parameters und dem Volumenanteil der zweiten Phase in dem Medium (M) mit dem ermittelten Streuungswert des zweiten elektrischen Parameters der Volumenanteil der zweiten Phase in dem Medium bestimmt wird.

8. Verfahren (1) nach Anspruch 7, **dadurch gekennzeichnet, dass** aus dem mit dem ersten elektrischen Parameter (E) des Mediums (M) ermittelte Volumenanteil (V%_P2) der zweiten Phase (P2) des Mediums (M) und aus dem mit dem zweiten elektrischen Parameter des Mediums ermittelte Volumenanteil der zweiten Phase des Mediums ein gemittelter Volumenanteil der zweiten Phase des Mediums berechnet wird.

9. Verfahren (1) nach einem der Ansprüche 1 bis 8, **dadurch gekennzeichnet, dass** der mit dem ersten elektrischen Parameter (E) des Mediums (M) ermittelte Volumenanteil (V%_P2) der zweiten Phase (P2) in dem Medium (M) und/oder der mit dem zweiten elektrischen Parameter des Mediums ermittelte Volumenanteil der zweiten Phase des Mediums (M) und/oder der gemittelte Volumenanteil der zweiten Phase (P2) in dem Medium (M) signalisiert wird, insbesondere in einem Speicher einer Steuer- und Auswerteeinheit (4) abgelegt wird und/oder angezeigt wird und/oder über eine Kommunikationsschnittstelle eines Messgeräts (2) an einen angeschlossenen Kommunikationspartner übermittelt wird.

10. Verfahren (1) nach einem der Ansprüche 1 bis 9, **dadurch gekennzeichnet, dass** bei Überschreiten eines ersten Grenzwertes durch den mit dem ersten elektrischen Parameter (E) des Mediums (M) ermittelten Volumenanteil (V%_P2) der zweiten Phase (P2) in dem Medium (M) und/oder bei Überschreiten eines zweiten Grenzwertes durch den mit dem zweiten elektrischen Parameter des Mediums ermittelten Volumenanteil der zweiten Phase des Mediums (M) und/oder bei Überschreiten eines dritten Grenzwertes durch den gemittelten Volumenanteil der zweiten Phase in dem Medium das Vorliegen einer Zwei-Phasen-Strömung signalisiert wird, insbesondere in einem Speicher einer Steuer- und Auswerteeinheit (4) abgelegt wird und/oder angezeigt wird und/oder über eine Kommunikationsschnittstelle eines Messgeräts (2) an einen angeschlossenen Kommunikationspartner übermittelt wird.

11. Verfahren (1) nach einem der Ansprüche 1 bis 10, **dadurch gekennzeichnet, dass** der mathematische Zusammenhang (f) zwischen dem statistischen Streuungsmaß (S) des ersten elektrischen Parameters (E) und dem Volumenanteil (V%_P2) der zweiten Phase des Mediums und/oder der mathematische Zusammenhang zwischen dem statistischen Streuungsmaß des zweiten elektrischen Parameters und dem Volumenanteil der zweiten Phase (P2) des Mediums (M) ermittelt wird, indem der Streuungswert (S(En)) des ersten elektrischen Parameters (E) und/oder der Streuungswert des zweiten elektrischen Parameters bei zumindest zwei verschiedenen aber bekannten Volumenanteilen (V%_P2) der zweiten Phase (P2) des Mediums (M) ermittelt werden, insbesondere wobei eine lineare Abhängigkeit zwischen dem statistischen Streuungsmaß des ersten elektrischen Parameters und dem Volumenanteil der zweiten Phase des Mediums und/oder zwischen dem statistischen Streuungsmaß des zweiten elektrischen Parameters und dem Volumenanteil der zweiten Phase des Mediums angenommen wird.

12. Verfahren (1) nach Anspruch 11, **dadurch gekennzeichnet, dass** mehr als zwei Stützstellen aufgenommen werden, also mehr als zwei Zusammenhänge zwischen dem statistischen Streuungsmaß (S) des ersten elektrischen Parameters (E) und dem Volumenanteil (V%_P2) der zweiten Phase (P2) des Mediums (M), und es wird als mathematischer Zusammenhang (f) zwischen dem statistischen Streuungsmaß (S) des elektrischen Parameters (E) und dem Volumenanteil (V%_P2) der zweiten Phase (P2) des Mediums (M) eine polynomiale Beschreibung des Zusammenhangs ermittelt oder eine Beschreibung des Zusammenhangs wird anhand einer Spline-Interpolationen ermittelt, insbesondere unter Optimierung der Beschreibungen der Zusammenhänge anhand der Minimierung eines Abweichungsmaßes.

13. Messgerät (2) mit einem zur Aufnahme eines Mediums (M) dienenden Messvolumens (6), wobei das Medium (M) eine erste Phase (P1) mit einem ersten Wert (E1) für einen ersten elektrischen Parameter (E) und zumindest zeitweilig eine zweite Phase (P2) mit einem zweiten Wert (E2) für den ersten elektrischen Parameter (E) aufweist, mit einer Steuer- und Auswerteeinheit (4), mit einem mit dem Medium (M) in Kontakt stehenden Elektrodenpaar (3), wobei die Steuer- und Auswerteeinheit (4) im Betriebszustand des Messgeräts (2) das Medium (M) über das Elektrodenpaar (3) mit einer Mehrzahl elektrischer Anregungssignale beaufschlagt und zu den elektrischen Anregungssignalen eine Mehrzahl entsprechender elektrischer Reaktionssignale erfasst, wobei aus der Mehrzahl an Anregungssignalen und der Mehrzahl an Reaktionssignalen eine Mehrzahl (En) von Werten für den ersten elektrischen Parameter (E) des Mediums (M) ermittelt wird,
**dadurch gekennzeichnet,**
**dass** die Steuer- und Auswerteeinheit (4) so ausgestaltet ist, dass das Messgerät (2) im Betrieb das Verfahren (1) nach einem der Ansprüche 1 bis 12 durchführt.

14. Messgerät (2) nach Anspruch 13, **dadurch gekennzeichnet, dass** das Messgerät (2) ein magnetisch-induktives Durchflussmessgerät ist mit einem Elektrodenpaar (3) zur Aufnahme einer in dem Medium (M) induzierten elektrischen Spannung, wobei das Elektrodenpaar (3) auch dazu verwendet wird, das Medium mit einer Mehrzahl elektrischer Anregungssignale zu beaufschlagen.

## Claims

1. Method (1) for determining the volume fraction (V%) of a phase of a multiphase medium (M), wherein the medium (M) has a first phase (P1) with a first value (E1) for a first electrical parameter (E) and, at least temporarily, a second phase (P2) with a second value (E2) for the first electrical parameter, wherein the medium (M) is impinged with a plurality of electrical excitation signals via a pair of electrodes (3) and a plurality of corresponding electrical reaction signals are captured for the electrical excitation signals, wherein a plurality of values (En) for the first electrical parameter (E) of the medium (M) is determined from the plurality of excitation signals and the plurality of reaction signals,
wherein a scattering value (S(En)) of a statistical scattering measure (S) is determined from the plurality of determined values (En) for the first electrical parameter (E) of the medium (M),
wherein the volume fraction (V%_P2) of the second phase (P2) in the medium (M) is determined using a mathematical relationship (f) between the statistical scattering measure (S) of the first electrical parameter (E) and a volume fraction (V%_P2) of the second phase (P2) in the medium (M) with the determined scattering value (S(En)) of the first electrical parameter (E),
wherein the first electrical parameter (E) of the medium (M) is the electrical conductivity (sigma) of the medium (M) and/or the absolute value of the impedance of the medium (M) and/or the phase angle of the impedance of the medium (M)

2. Method (1) according to claim 1, **characterized in that** the excitation signal is an electrical voltage or an electrical current, and that the electrical response signal is an electrical current or an electrical voltage, in particular wherein the excitation signal is a harmonic oscillation with an excitation frequency.

3. Method (1) according to claim 1 or 2, **characterized in that** the impingement of the medium (M) with the electrical excitation signals takes place in a time domain in which no other measured value acquisition is carried out in the medium (M), that, in particular in the case of a magnetic-inductive measuring device, no magnetic-inductive flow measurement is carried out, then in particular after a switching of the polarity of the magnetic field (B), in particular in the time domain of a non-stationary magnetic field (B).

4. Method (1) according to any one of claims 1 to 3, **characterized in that** the standard deviation is calculated as the statistical scattering measure (S).

5. Method (1) according to any one of claims 1 to 4, **characterized in that** the electrical conductivity is determined from the real part of the impedance of the medium (M), taking into account the diameter of a measurement volume (6) for the medium (M).

6. Method (1) according to any one of claims 1 to 5, **characterized in that** the determined scattering value (S(En)) of the first electrical parameter is normalized to the absolute value of the first electrical parameter (E), in particular to an absolute mean value of the first electrical parameter (E) calculated from the plurality of determined values for the first electrical parameter (E) of the medium (M).

7. Method (1) according to any one of claims 1 to 6, **characterized in that** a plurality of values for a second electrical parameter of the medium (M) is determined from the plurality of excitation signals and the plurality of reaction signals, that a scattering value of a statistical scattering measure is determined from the plurality of determined values for the second electrical parameter of the medium (M), and that, using a mathematical relationship between the statistical scattering measure of the second electrical parameter and the volume fraction of the second phase in the medium (M), the volume fraction of the second phase in the medium is determined using the determined scattering value of the second electrical parameter.

8. Method (1) according to claim 7, **characterized in that** an averaged volume fraction of the second phase of the medium is calculated from the volume fraction (V%_P2) of the second phase (P2) of the medium (M) determined with the first electrical parameter (E) of the medium (M) and from the volume fraction of the second phase of the medium determined with the second electrical parameter of the medium.

9. Method (1) according to any one of claims 1 to 8, **characterized in that** the volume fraction (V%_P2) of the second phase (P2) in the medium (M) determined with the first electrical parameter (E) of the medium (M) and/or the volume fraction of the second phase of the medium (M) determined with the second electrical parameter of the medium and/or the averaged volume fraction of the second phase (P2) in the medium (M) is signaled, in particular is stored in a memory of a control and evaluation unit (4) and/or is displayed and/or is transmitted to a connected communication partner via a communication interface of a measuring device (2).

10. Method (1) according to any one of claims 1 to 9, **characterized in that** when a first limit value is exceeded, the presence of a two-phase flow is signalled by the volume fraction (V%_P2) of the second phase (P2) in the medium (M) determined using the first electrical parameter (E) of the medium (M) and/or when a second limit value is exceeded, the volume fraction of the second phase of the medium (M) determined using the second electrical parameter of the medium and/or when a third limit value is exceeded, the presence of a two-phase flow is signalled by the averaged volume fraction of the second phase in the medium, in particular is stored in a memory of a control and evaluation unit (4) and/or is displayed and/or is transmitted to a connected communication partner via a communication interface of a measuring device (2).

11. Method (1) according to any one of claims 1 to 10, **characterized in that** the mathematical relationship (f) between the statistical scattering measure (S) of the first electrical parameter (E) and the volume fraction (V%_P2) of the second phase of the medium and/or the mathematical relationship between the statistical scattering measure of the second electrical parameter and the volume fraction of the second phase (P2) of the medium (M) is determined **in that** the scattering value (S(En)) of the first electrical parameter (E) and/or the scattering value of the second electrical parameter are determined for at least two different but known volume fractions (V%_P2) of the second phase (P2) of the medium (M), in particular wherein a linear dependence is assumed between the statistical scattering measure of the first electrical parameter and the volume fraction of the second phase of the medium and/or between the statistical scattering measure of the second electrical parameter and the volume fraction of the second phase of the medium.

12. Method (1) according to claim 11, **characterized in that** more than two supporting points are recorded, i.e. more than two correlations between the statistical scattering measure (S) of the first electrical parameter (E) and the volume fraction (V%_P2) of the second phase (P2) of the medium (M), and a polynomial description of the relationship is determined as the mathematical relationship (f) between the statistical scattering measure (S) of the electrical parameter (E) and the volume fraction (V%_P2) of the second phase (P2) of the medium (M), or a description of the relationship is determined using spline interpolations, in particular by optimizing the descriptions of the relationships using the minimization of a deviation measure.

13. Measuring device (2) with a measurement volume (6) serving to accommodate a medium (M), wherein the medium (M) has a first phase (P1) with a first value (E1) for a first electrical parameter (E) and, at least temporarily, a second phase (P2) with a second value (E2) for the first electrical parameter (E), with a control and evaluation unit (4), with a pair of electrodes (3) in contact with the medium (M), wherein the control and evaluation unit (4), in the operating state of the measuring device (2), impinges a plurality of electrical excitation signals on the medium (M) via the pair of electrodes (3) and captures a plurality of corresponding electrical response signals for the electrical excitation signals, wherein a plurality (En) of values for the first electrical parameter (E) of the medium (M) is determined from the plurality of excitation signals and the plurality of response signals,
**characterized in**
**that** the control and evaluation unit (4) is designed such that the measuring device (2) carries out the method (1) according to any one of claims 1 to 12 during operation.

14. Measuring device (2) according to claim 13, **characterized in that** the measuring device (2) is a magnetic-inductive flowmeter with a pair of electrodes (3) for recording an electrical voltage induced in the medium (M), wherein the pair of electrodes (3) is also used to impinge a plurality of electrical excitation signals on the medium.

## Revendications

1. Procédé (1) pour déterminer la fraction volumique (V%) d'une phase d'un milieu polyphasique (M), le milieu (M) comportant une première phase (P1) avec une première valeur (E1) pour un premier paramètre électrique (E) et au moins temporairement une deuxième phase (P2) avec une deuxième valeur (E2) pour le premier paramètre électrique, une pluralité de signaux d'excitation électriques étant appliqués au milieu (M) par l'intermédiaire d'une paire d'électrodes (3) et une pluralité de signaux de réaction électriques correspondant aux signaux d'excitation électriques étant détectés, une pluralité de valeurs (En) pour le premier paramètre électrique (E) du milieu (M) étant déterminée à partir de la pluralité de signaux d'excitation et de la pluralité de signaux de réaction,
une valeur de dispersion (S(En)) d'une mesure de dispersion statistique (S) étant déterminée à partir de la pluralité de valeurs déterminées (En) pour le premier paramètre électrique (E) du milieu (M),
la fraction volumique (V%_P2) de la deuxième phase (P2) dans le milieu (M) étant déterminée à l'aide d'une relation mathématique (f) entre la mesure de dispersion statistique (S) du premier paramètre électrique (E) et une fraction volumique (V%_P2) de la deuxième phase (P2) dans le milieu (M) ayant la valeur de dispersion déterminée (S(En)) du premier paramètre électrique (E), et
le premier paramètre électrique (E) du milieu (M) étant la conductivité électrique (sigma) du milieu (M) et/ou le module de l'impédance du milieu (M) et/ou l'angle de phase de l'impédance du milieu (M).

2. Procédé (1) selon la revendication 1, **caractérisé en ce que** le signal d'excitation est une tension électrique ou un courant électrique et **en ce que** le signal de réaction électrique est un courant électrique ou une tension électrique, le signal d'excitation étant en particulier une oscillation harmonique présentant une fréquence d'excitation.

3. Procédé (1) selon la revendication 1 ou 2, **caractérisé en ce que** l'application au milieu (M) des signaux d'excitation électriques est effectuée dans une plage temporelle dans laquelle aucune autre détection de valeur de mesure n'est effectuée dans le milieu (M), **en ce qu'**en particulier, dans le cas d'un appareil de mesure électromagnétique, aucune mesure de débit électromagnétique n'est effectuée, en particulier après une inversion de polarité du champ magnétique (B), en particulier dans la plage temporelle d'un champ magnétique non stationnaire (B).

4. Procédé (1) selon l'une quelconque des revendications 1 à 3, **caractérisé en ce que** l'écart-type est calculé en tant que mesure de dispersion statistique (S).

5. Procédé (1) selon l'une quelconque des revendications 1 à 4, **caractérisé en ce que** la conductivité électrique est déterminée à partir de la partie réelle de l'impédance du milieu (M) en tenant compte du diamètre d'un volume de mesure (6) pour le milieu (M).

6. Procédé (1) selon l'une quelconque des revendications 1 à 5, **caractérisé en ce que** la valeur de dispersion déterminée (S(En)) du premier paramètre électrique est normalisée par rapport à la valeur absolue du premier paramètre électrique (E), en particulier par rapport à une valeur moyenne absolue du premier paramètre électrique (E) calculée à partir de la pluralité de valeurs déterminées pour le premier paramètre électrique (E) du milieu (M).

7. Procédé (1) selon l'une quelconque des revendications 1 à 6, **caractérisé en ce qu'**une pluralité de valeurs pour un deuxième paramètre électrique du milieu (M) sont déterminées à partir de la pluralité de signaux d'excitation et de la pluralité de signaux de réaction, **en ce qu'**une valeur de dispersion d'une mesure de dispersion statistique est déterminée à partir de la pluralité de valeurs déterminées pour le deuxième paramètre électrique du milieu (M), et **en ce que** la fraction volumique de la deuxième phase dans le milieu est déterminée à l'aide d'une relation mathématique entre la mesure de dispersion statistique du deuxième paramètre électrique et la fraction volumique de la deuxième phase dans le milieu (M) avec la valeur de dispersion déterminée du deuxième paramètre électrique.

8. Procédé (1) selon la revendication 7, **caractérisé en ce qu'**une fraction volumique moyenne de la deuxième phase du milieu est calculée à partir de la fraction volumique (V%_P2) de la deuxième phase (P2) du milieu (M) déterminée avec le premier paramètre électrique (E) du milieu (M) et à partir de la fraction volumique de la deuxième phase du milieu déterminée avec le deuxième paramètre électrique du milieu.

9. Procédé (1) selon l'une quelconque des revendications 1 à 8, **caractérisé en ce que** la fraction volumique (V%_P2) de la deuxième phase (P2) dans le milieu (M), déterminée avec le premier paramètre électrique (E) du milieu (M) et/ou la fraction volumique de la deuxième phase du milieu (M), déterminée avec le deuxième paramètre électrique du milieu et/ou la fraction volumique moyenne de la deuxième phase (P2) dans le milieu (M) est signalée, en particulier stockée dans une mémoire d'une unité de commande et d'évaluation (4) et/ou affichée et/ou transmise à un partenaire de communication connecté par l'intermédiaire d'une interface de communication d'un appareil de mesure (2).

10. Procédé (1) selon l'une quelconque des revendications 1 à 9, **caractérisé en ce qu'**en cas de dépassement d'une première valeur limite par la fraction volumique (V%_P2) de la deuxième phase (P2) dans le milieu (M), déterminée avec le premier paramètre électrique (E) du milieu (M) et/ou **en ce qu'**en cas de dépassement d'une deuxième valeur limite par la fraction volumique de la deuxième phase du milieu (M), déterminée avec le deuxième paramètre électrique du milieu et/ou **en ce qu'**en cas de dépassement d'une troisième valeur limite par la fraction volumique moyenne de la deuxième phase dans le milieu, la présence d'un écoulement biphasique est signalée, en particulier stockée dans une mémoire d'une unité de commande et d'évaluation (4) et/ou affichée et/ou transmise à un partenaire de communication connecté par l'intermédiaire d'une interface de communication d'un appareil de mesure (2).

11. Procédé (1) selon l'une quelconque des revendications 1 à 10, **caractérisé en ce que** la relation mathématique (f) entre la mesure de dispersion statistique (S) du premier paramètre électrique (E) et la fraction volumique (V%_P2) de la deuxième phase du milieu et/ou la relation mathématique entre la mesure de dispersion statistique du deuxième paramètre électrique et la fraction volumique de la deuxième phase (P2) du milieu (M) est déterminée par détermination de la valeur de dispersion (S(En)) du premier paramètre électrique (E) et/ou de la valeur de dispersion du deuxième paramètre électrique pour au moins deux fractions volumiques (V%_P2) de la deuxième phase (P2) du milieu (M), différentes mais connues, une dépendance linéaire étant en particulier supposée entre la mesure de dispersion statistique du premier paramètre électrique et la fraction volumique de la deuxième phase du milieu et/ou entre la mesure de dispersion statistique du deuxième paramètre électrique et la fraction volumique de la deuxième phase du milieu.

12. Procédé (1) selon la revendication 11, **caractérisé en ce que** plus de deux points d'appui sont acquis, c'est-à-dire plus de deux relations entre la mesure de dispersion statistique (S) du premier paramètre électrique (E) et la fraction volumique (V%_P2) de la deuxième phase (P2) du milieu (M), et **en ce qu'**une description polynomiale de la relation est déterminée en tant que relation mathématique (f) entre la mesure de dispersion statistique (S) du paramètre électrique (E) et la fraction volumique (V%_P2) de la deuxième phase (P2) du milieu (M) ou **en ce qu'**une description de la relation est déterminée sur la base d'une interpolation spline, en particulier en optimisant les descriptions des relations sur la base de la minimisation d'une mesure d'écart.

13. Appareil de mesure (2) comprenant un volume de mesure (6) destiné à recevoir un milieu (M), le milieu (M) comportant une première phase (P1) avec une première valeur (E1) pour un premier paramètre électrique (E) et au moins temporairement une deuxième phase (P2) avec une deuxième valeur (E2) pour le premier paramètre électrique (E), comprenant une unité de commande et d'évaluation (4), une paire d'électrodes (3) en contact avec le milieu (M), l'unité de commande et d'évaluation (4), dans l'état de fonctionnement de l'appareil de mesure (2), appliquant une pluralité de signaux d'excitation électriques au milieu (M) par l'intermédiaire de la paire d'électrodes (3) et détectant une pluralité de signaux de réaction électriques correspondant aux signaux d'excitation électriques, une pluralité de valeurs (En) pour le premier paramètre électrique (E) du milieu (M) étant déterminée à partir de la pluralité de signaux d'excitation et de la pluralité de signaux de réaction, **caractérisé**
**en ce que** l'unité de commande et d'évaluation (4), en fonctionnement, est configurée de telle sorte que l'appareil de mesure (2), en fonctionnement, mette en œuvre un procédé (1) selon l'une quelconque des revendications 1 à 12.

14. Dispositif de mesure (2) selon la revendication 13, **caractérisé en ce que** l'appareil de mesure (2) est un débitmètre électromagnétique comprenant une paire d'électrodes (3) destinées à recevoir une tension électrique induite dans le milieu (M), la paire d'électrodes (3) étant également utilisée pour appliquer plusieurs signaux d'excitation électriques au milieu.
